# EUROPEAN PATENT APPLICATION

(11) **EP 2 544 460 A2**
(43) Date of publication of application: **09.01.2013**
(21) Application number: 12161095.0
(22) Date of filing: 23.03.2012
(51) Int. Cl.: H04N 13/04, H04N 21/422, H04N 21/442

(54) **Method for controlling display apparatus using brain wave and display apparatus thereof**

(30) Priority: 07.07.2011 KR 20110067345
(71) Applicant: Samsung Electronics Co., Ltd., Suwon-si, Gyeonggi-do, 443-742 (KR)
(72) Inventor: Jo, Chan-young, Gyeonggi-do (KR)
(74) Representative: Fearnside, Andrew Simon

(57) **Abstract**

A display apparatus is controlled using brain waves. Brain wave information of a user is obtained and compared with pre-stored brain wave information previously mapped to control signals. A control signal corresponding to the brain wave information is generated, and the display apparatus is controlled according to the generated control signal. As a result, a user may control a display apparatus more conveniently and the display apparatus may provide various beneficial applications that take advantage of the capture of the brain wave information.

## Description

### CROSS-REFERENCE TO RELATED APPLICATIONS

This application claims priority from Korean Patent Application No. 10-2011-0067345, filed in the Korean Intellectual Property Office on July 7, 2011, the disclosure of which is incorporated herein by reference.

### BACKGROUND

### Field

Methods and apparatuses consistent with exemplary embodiments herein relate to a method for controlling a display apparatus using a brain wave, and a display apparatus using the same, and more particularly, to a method for controlling a display apparatus which generates a display control signal using a brain wave and a display apparatus using the same.

### Description of the Related Art

A smart television (TV) now has various functions, and its interface has become multifunctional and more sophisticated.

For example, methods have been developed in which a user controls a display apparatus by touching the display, using a remote controller via wireless communication such as Bluetooth, or using a voice recognition or an image analysis technique.

Such interfaces improve user convenience since they allow a user to control an apparatus remotely in a more convenient way, and also provide various inputs and outputs. In this regard, consumers now consider as important not only the functions of a display apparatus itself, but also the interfaces to control the display apparatus.

Recently, much research on human brain waves has been conducted to find out how a human being thinks and feels. Specifically, a brain wave is what controls not only movement of muscles around the eyes, but also decision-making and feeling. Thus, brain waves measured may be catalogued in a database, and based on the contents of the database, a brain wave can be analyzed to gauge someone's thinking and feeling. Recently, there was an experiment to input a text message using only brain waves, and the result was successful.

As it becomes more possible to detect people's thoughts and feelings using a brain waves, the development of a method for controlling a display apparatus using a brain wave also becomes possible.

### SUMMARY

Exemplary embodiments provide a method for controlling a display apparatus which compares brain wave information, acquired from a user watching the display apparatus, with pre-stored brain wave information and generates a control signal corresponding to the brain wave information, and a display apparatus using the same.

According to the present invention there is provided an apparatus and method as set forth in the appended claims. Other features of the invention will be apparent from the dependent claims, and the description which follows.

According to an aspect of an exemplary embodiment, there is provided a method for controlling a display apparatus including obtaining brain wave information of a user, comparing the brain wave information with pre-stored brain wave information, and generating a control signal corresponding to the pre-stored brain wave information, and controlling a display apparatus according to the generated control signal.

The display apparatus may be a 3D display apparatus, and the obtaining may include obtaining a brain wave of the user from a brain wave detector attached to 3D glasses which are operating in cooperation with the 3D apparatus.

The method may further include, in response to user input, displaying a GUI for setting the display apparatus, and the generating may include generating a control signal for manipulating a setting of the GUI according to the obtained brain wave information.

If the GUI is to adjust the volume level, the generating may include generating a control signal to increase the volume level when it is detected that first brain wave information is included in the obtained brain wave information, and generating a control signal to decrease the volume level when it is detected that second brain wave information is included in the obtained brain wave information.

If the GUI is to change channels, the generating may include generating a control signal to change channels in a first direction when it is detected that third brain wave information is included in the obtained brain wave information, and generating a control signal to change channels in a direction opposite to the first direction when it is detected that fourth brain wave information is included in the obtained brain wave information.

The pre-stored brain wave information may be mapped to a control signal of the display apparatus and may be stored separately for each of a plurality of users.

The method may further include storing the obtained brain wave information and transmitting the stored brain wave information to an external health check-up system to perform a health check-up of the user, using the transmitted brain wave information.

The method may further include obtaining a body-related information using the obtained brain wave information and displaying the obtained body-related information.

The method may further include controlling the selection and the volume level of music of a meditation application provided by the display apparatus based on the obtained brain wave information.

The method may further include determining a user content preference based on the obtained brain wave information, and providing a suggestion as to other contents based on the determination results.

According to an aspect of another exemplary embodiment, there is provided a display apparatus including a display unit, a storage unit which maps brain wave information to a control signal of the display apparatus and stores the information, a receiving unit which receives brain wave information of a user, a comparison unit which compares the obtained brain wave information with brain wave information pre-stored in the storage unit, and a control unit which generates a control signal corresponding to the brain wave information obtained based on the comparison result and controls a display apparatus according to the generated control signal.

The display apparatus may be a 3D display apparatus, and the receiving unit may obtain a brain wave of the user from a brain wave detector attached to 3D glasses which are operating in cooperation with the 3D display apparatus.

The apparatus may further include a GUI generating unit which generates a GUI for setting the display apparatus in response to a user input, and the control unit may generate a control signal for manipulating a setting of the GUI according to the obtained brain wave information.

When the GUI is to adjust volume, the control unit may generate a control signal to increase the volume level when it is determined that first brain wave information is detected in the obtained brain wave information, and generate a control signal to decrease the volume level when it is detected that second brain wave information is included in the obtained brain wave information.

If the GUI is to change channels, the control unit may generate a control signal to change channels in a first direction when it is detected that third brain wave information is included in the obtained brain wave information, and generate a control signal to change channels in a direction opposite to the first direction when it is detected that fourth brain wave information is included in the obtained brain wave information.

The pre-stored brain wave information may be stored separately for each of a plurality of users.

The apparatus may further include a communication unit which communicates with an external network, and the storage unit may store brain wave information obtained from the brain wave information obtaining unit, and the control unit may control the communication unit to transmit the stored brain wave information to an external health check-up system to perform a health check-up of the user based on the transmitted brain wave.

The control unit may control the display unit to obtain a body-related information using the obtained brain wave information and to display the obtained body-related information.

The control unit may control the selection and the volume level of music of a meditation application provided by the display apparatus using the obtained brain wave information.

The control unit may determine a user content preference using the obtained brain wave information, and other and may recommend contents to the user, based on the determination results.

### BRIEF DESCRIPTION OF THE DRAWINGS

The above and/or other aspects of the present disclosure will be more apparent by describing certain exemplary embodiments with reference to the accompanying drawings, in which:
FIG. 1 is a schematic block diagram illustrating a display system to control a display apparatus using brain wave information according to an exemplary embodiment;
FIG. 2 is a block diagram illustrating a brain wave detector according to an exemplary embodiment;
FIG. 3 is a block diagram illustrating a display apparatus according to an exemplary embodiment;
FIGS. 4 and 5 are views illustrating a graphic user interface (GUI) for controlling a display apparatus using brain wave information according to an exemplary embodiment; and
FIG. 6 is a flowchart to explain a method for controlling a display apparatus using a brain wave according to an exemplary embodiment.

### DETAILED DESCRIPTION

Certain exemplary embodiments are described in greater detail below with reference to the accompanying drawings.

In the following description, like drawing reference numerals are used for like elements. The particular details set forth in the description, such as detailed construction and elements, are provided to assist in a comprehensive understanding of exemplary embodiments. However, the teachings represented by the exemplary embodiments the particular details do not necessarily require. Also, well-known functions or constructions are not described in detail since they might needlessly obscure the inventive concept.

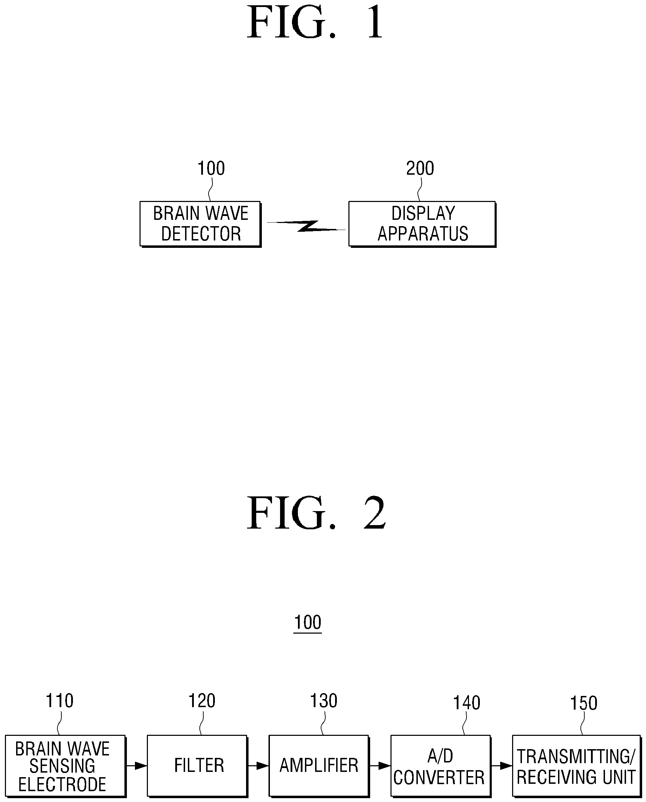

FIG. 1 is a schematic block diagram illustrating a display system to control a display apparatus 200, using brain wave information, according to an exemplary embodiment. As illustrated in FIG. 1, the display system includes a brain wave detector 100 and the display apparatus 200.

The brain wave detector 100 detects a brain wave of a user using a plurality of brain wave sensing electrodes. In addition, the brain wave detector 100 processes brain waves detected from the plurality of sensing electrodes and transmits them to an external display apparatus 200. In particular, if the display apparatus 200 is a three-dimensional (3D) display apparatus, the brain wave detector 100 may be provided on a pair of 3D glasses that cooperate with the 3D display.

The display apparatus 200 obtains brain wave information of a user using the display apparatus 200, from the external brain wave detector 100.

If brain wave information is received from the external brain wave detector 100, the display apparatus 200 processes the received brain wave information in a matrix form or a histogram, form and stores the processed information in a storage unit, and compares the processed information with pre-stored brain wave information to generate a control signal.

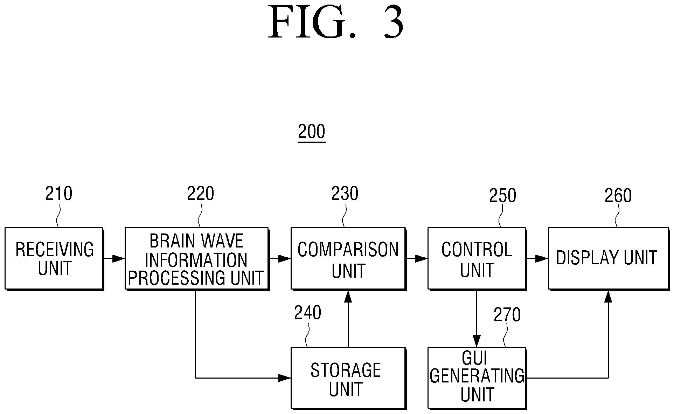

In this case, the storage unit 240 of the display apparatus 200 performs and stores the mapping between specific brain wave information and a specific control signal (see Fig. 3). For example, the storage unit 240 performs and stores a mapping between the first brain wave information and a control signal of "enter". In this case, the storage unit 240 may perform and store a mapping between specific brain wave information and a specific control signal for each user. For example, the mapping between the first brain wave information and a control signal of "enter" may be performed for a user A, and the mapping between the second brain wave information and a control signal of "enter" may be performed for a user B, since each user may output a different brain wave with respect to a specific control signal.

The display apparatus 200 compares received brain wave information and pre-stored brain wave information and generates a control signal corresponding to the brain wave information received from the brain wave detector 100. For example, if it is determined that the brain wave information received from the brain wave detector 100 contains the first bran wave information according to the comparison result, the display apparatus 200 generates a control signal of "enter" which is mapped with the first brain wave information.

In addition, the display apparatus 200 may provide various services and applications using the brain wave information received from the brain wave detector 100. For example, the display apparatus 200 may regularly transmit brain wave information to an external health check-up system via a communication unit (not shown), and may provide various applications such as a health check-up application, a meditation application and a recommended contents application. This will be explained in detail later.

Meanwhile, the display apparatus 200 according to an exemplary embodiment may be a 3D display apparatus which is interlocked with a pair of 3D glasses to which the brain wave detector 100 is attached.

As described above, since the display apparatus 200 is controlled using brain wave information, a user may more conveniently control the display apparatus and the display apparatus may provide various related applications.

Hereinafter, the brain wave detector 100 and the display apparatus 200 will be explained in more detail using FIGS. 2 and 3.

FIG. 2 is a block diagram illustrating the brain wave detector 100 according to an exemplary embodiment. As illustrated in FIG. 2, the brain wave detector 100 includes a brain wave sensing electrode 110, a filter 120, an amplifier 130, an A/D converter 140, and a transmitting/receiving unit 150.

The brain wave sensing electrode 110 senses a user's brain wave. In particular, if the brain wave detector 100 is included in 3D glasses, the brain wave sensing electrode 110 may be disposed on the arm of the 3D glasses or on the part contacting the user's forehead.

In this case, the brain wave sensing electrode 110 includes an alpha electrode detecting a brain wave in the range of 8∼13Hz, a beta electrode detecting a brain wave in the range of 13∼30Hz, a gamma electrode detecting a brain wave in the range of 30∼50Hz, a delta electrode detecting a brain wave in the range of 1∼4Hz, or a theta electrode detecting a brain wave in the range of 4∼8Hz.

The filter 120 filters the brain wave sensed by the brain wave sensing electrode 110 and outputs the filtered brain wave to the amplifier 130. In this case, the filter 120 may be realized as a low pass filter (LPF).

The amplifier 130 amplifies the brain wave filtered by the filter 120 and outputs the amplified brain wave to the A/D converter 140.

The A/D converter 140 converts the amplified brain wave information from an analog signal to a digital signal.

Subsequently, the transmitting/receiving unit 150 transmits the brain wave, which is in the form of the digital signal, to the external display apparatus 200. In this case, the transmitting/receiving unit 150 may be realized by modules such as a Wi-Fi module and an IR module.

FIG. 3 is a block diagram illustrating the display apparatus 200 according to an exemplary embodiment. As illustrated in FIG. 3, the display apparatus 200 includes a receiving unit 210, a brain wave information processing unit 220, a comparison unit 230, a storage unit 240, a control unit 250, a display unit 260, and a GUI generating unit 270.

The receiving unit 210 receives a brain wave which is transmitted from the external brain wave detector 100. In this case, the receiving unit 210 may be realized using a Wi-Fi module or an IR module, in the same way as the transmitting/receiving unit 150 is realized.

The brain wave information processing unit 220 processes the brain wave information, output from the receiving unit 210, in the form of histogram or matrix. In addition, the brain wave information processing unit 220 outputs the processed brain wave information to the storage unit 240 and/or the comparison unit 230.

The comparison unit 230 compares the brain wave information which is currently received from the external brain wave detector 100 with the brain wave information which is pre-stored in the storage unit 240 and outputs the comparison result to the control unit 250.

Specifically, the comparison unit 230 conducts a search to determine whether there is brain wave information in the storage unit 240 corresponding to the brain wave information which is currently received from the external brain wave detector 100. If it is determined that there is stored brain wave information corresponding to the brain wave information which is currently received, the comparison unit 230 outputs the corresponding pre-stored brain wave information to the control unit 250. In this case, the currently received brain wave information and the pre-stored brain wave information are determined to be identical to each other not only when the currently received brain wave information and the pre-stored brain wave information are completely consistent with each other but also when the differences are within a predetermined error range.

The storage unit 240 performs a mapping of brain wave information, in the form of a histogram or a matrix with a control signal and stores the mapping information. For example, the storage unit 240 may perform mapping between brain wave information and control signals as in table 1 below and store the information.

**[Table 1]**

| Brain wave information | Control command |
|---|---|
| First brain wave information | enter |
| Second brain wave information | increase |
| Third brain wave information | decrease |
| Fourth brain wave information | cancel |

That is, the storage unit 240 maps the first brain wave information to a control signal of "enter" and stores the information, maps the second brain wave information to "increase" and stores the information, maps the third brain wave information to "decrease" and stores the information, and maps the fourth brain wave information to "cancel" and stores the information. However, the above control commands of "enter", "increase", "decrease", and "cancel" are only examples, and other control commands such as "turn off power" and "convert to 3D mode" may also be included. In Table 1, the first brain wave information may be understood to constitute first stored brain wave entry. Likewise, the second, third, and fourth brain wave information may be understood to constitute second, third, and fourth stored brain wave entries. Here, the term "entry" does not mean that the various stored brain wave entries must be kept in a table; rather, the various stored brain wave entries can be stored in any appropriate concrete manner.

The storage unit 240 may store brain wave information and control signals differently for each user. For example, the storage unit 240 may map the first brain wave information to a control signal of "enter" and store the information for the first user, and map the fifth brain wave information to a control signal of "enter" and store the information for the second user.

The display unit 260 outputs externally received image data under the control of the control unit 250. In addition, the display unit 250 may also display a GUI generated by the GUI generating unit 270 in the form of an on-screen display (OSD).

The GUI generating unit 270 generates a GUI under the control of the control unit 250. In particular, the GUI generating unit 270 may generate a GUI for controlling the display apparatus 100 using the brain wave received from the external brain wave detector 100. This will be explained in greater detail later.

The control unit 250 may control the overall operation of the display apparatus 200 according to a user's command input from a user input unit (not shown). In particular, the control unit 250 may control the display apparatus 200 by generating a control signal according to the brain wave information received from the external brain wave detector 100.

Specifically, the control unit 250 outputs a control signal corresponding to the received brain wave information based on a comparison result from the comparison unit 240. For example, the control unit 250 generates a control signal of "enter" if the received brain wave information includes the first brain wave information. Likewise, the control unit 250 may generate a control signal of "increase" if the received brain wave information includes the second brain wave information, a control signal "decrease" if it includes the third brain wave information, and a control signal of "cancel" if it includes the fourth brain wave information. In addition, the control unit 250 controls the overall operation of the display apparatus 200 according to the generated control signal.

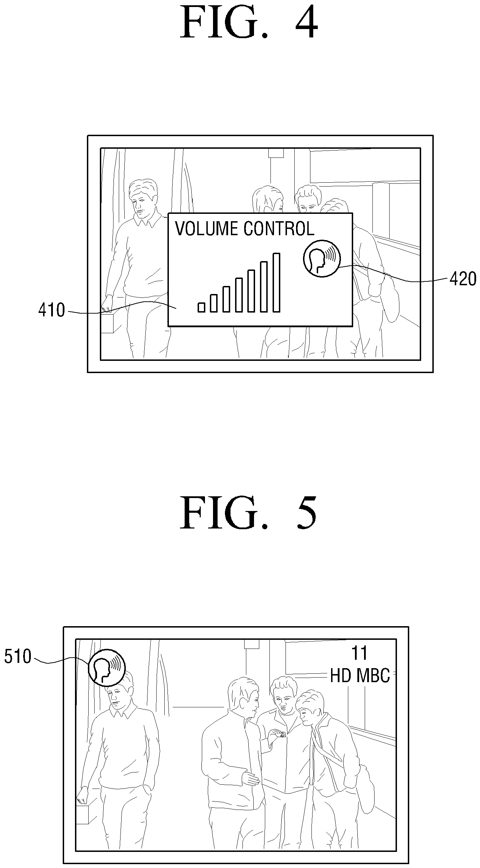

The control unit 250 may also control the GUI generating unit to generate a GUI which controls the display apparatus 200 using the received brain wave information. This will be explained with reference to FIGS. 4 and 5.

If a specific command is input from a user, the control unit 250 may generate and display a GUI 410, as illustrated in Fig. 4, which adjusts volume using brain wave information. If the second brain wave information is received after the GUI for adjusting volume is generated, the control unit 250 generates a control signal including a command for increasing the volume. On the other hand, if the third brain wave information is received, the control unit 250 generates a control signal including a command for decreasing the volume. If the fourth brain wave information is received , the control unit 250 generates a control signal of "confirm" and controls a speaker (not shown) to output volume corresponding to the fourth brain wave information. In this case, the control unit 250 may control the GUI generating unit 270 and the display unit 260 to generate and display a brain wave control icon such as 420 including information that the volume adjustment GUI 410 is controlled by a brain wave.

In another exemplary embodiment, if a specific command is input from a user, the control unit 250 may display a GUI 510 for changing channels using brain wave information as illustrated in FIG. 5. In this case, if the second brain wave information is received after the GUI 510 for changing channels is displayed, the control unit 250 generates a control signal including a command for changing channels by increasing channel numbers, and if the third brain wave information is received, the control unit 250 generates a control signal including a command for changing channels by decreasing channel numbers. If the fourth brain wave information is received, the control unit 250 generates a control signal of "confirm" and controls a tuner (not shown) to output a channel corresponding to the fourth brain wave information.

In addition, the control unit 250 may perform various service functions and applications using received brain wave information.

For example, the control unit 250 stores received brain wave information in the storage unit 240. In addition, the control unit 250 may control the communication unit (not shown) to regularly transmit the stored brain wave information to an external health check-up system (for example, once a month). Accordingly, a user may be provided with a health check-up service using brain waves sent via the display apparatus 200.

In another exemplary embodiment, the control unit 250 may determine a user's body-related information, using the received brain wave information, and display the less-obtained body-related information. Specifically, since brain wave information reflects body-related information (for example, heartbeat, blood pressure, and body temperature), the control unit 250 may calculate body-related information using the received brain wave information and output to the display unit 260 the calculated body-related information.

In one exemplary embodiment,, when the display apparatus 100 is used as a monitor for a treadmill, the control unit 250 may display the body-related information, or other body-related information such as the user's heartbeat, blood pressure, or body temperature during exercise based on the received brain wave information and on a real-time basis. The operation of the treadmill may be controlled based on such information. For example, if the heartbeat calculated based on received brain wave information exceeds a predetermined value, the control unit 250 may generate a control signal including a control command to reduce the speed of the treadmill.

In another exemplary embodiment, the control unit 250 may provide a meditation application using obtained brain wave information. Specifically, the control unit 250 may adjust the type of music and the volume of music of a meditation application using the received brain wave information. If it is determined that a user is highly concentrating on the meditation, the control unit 250 may exercise control so as to turn off the meditation music gradually. If it is determined that a user is not concentrating enough on the meditation, the control unit 250 may exercise control so as to change the meditation music or to increase the volume of the music.

In another exemplary embodiment, the control unit 250uses received brain wave information to detect whether a user has a preference for particular content, taking advantage of the fact that the brain wave information indicates people's feelings. More particularly, if a positive feeling is indicated by the content of the brain wave information while a user is watching particular content, then the control unit 250 concludes that the user prefers the particular content. That is to say, the control unit monitors the received brain wave information, and stores an indication of what content is being displayed when it interprets the brain wave response as being favorable. The control unit 250 repeats this kind of operation to determine what type of content that the user prefers. Furthermore, the control unit 250 may recommend contents to the viewer based on the types of content that were being displayed when the received brain wave information indicated a positive feeling. In other words, the control unit 250 may output a suggestion of content that has a characteristic in common with the content previously determined to be favorable content.

As described above, by controlling the display apparatus 200 using a brain wave, a user may more conveniently control a display apparatus and the display apparatus may provide various helpful and beneficial applications.

Hereinafter, a method for controlling the display apparatus 200 using brain wave information will be explained in greater detail.

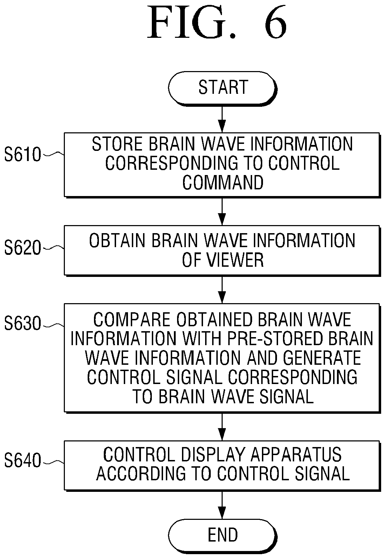

FIG. 6 is a flowchart to explain a method for controlling the display apparatus 200 using a brain wave according to an exemplary embodiment.

First of all, the display apparatus 200 stores brain wave information corresponding to a control command (S610). In this case, the display apparatus 200 may store brain wave information corresponding to a control signal differently for each user. In addition, the display apparatus 200 may store brain wave information corresponding to a control signal in the form of a histogram or a matrix.

In addition, the display apparatus 200 obtains brain wave information of a user from the external brain wave detector 100 (S620). In this case, the display apparatus 200 may obtain brain wave information wirelessly using a Wi-Fi module or an IR module. In addition, the display apparatus 200 may process the received brain wave information in the form of a histogram or a matrix.

The display apparatus 200 compares the received brain wave information with pre-stored brain wave information, and generates the control signal that corresponds to the matched, pre-stored brain wave signal (S630). Specifically, the display apparatus 200 conducts a search to determine whether there is previously stored brain wave information of the user which matches the received brain wave information. If it is determined that there is previously stored brain wave information of the user which matches the received brain wave information, the display apparatus 200 generates the control signal which has been previously mapped with the matched brain wave information.

Subsequently, the display apparatus 200 controls the display apparatus 200 according to the output control signal.

Accordingly, a user may control the display apparatus 200 and a more intuitive and convenient manner by controlling the display apparatus 200 using a brain wave, without the need for further inputs.

Although a few exemplary embodiments have been shown and described, it will be appreciated by those familiar with this field that changes may be made without departing from the principles and spirit of the inventive concept, the scope of which is to be understood in the light of the claims and their equivalents.

## Claims

1. A method for controlling a display apparatus, the method comprising:
obtaining brain wave information of a user;
comparing the brain wave information with pre-stored brain wave information and generating a control signal corresponding to the brain wave information; and
controlling a display apparatus according to the generated control signal.

2. The method as claimed in claim 1, wherein the display apparatus is a 3D display apparatus,
wherein the obtaining comprises obtaining a brain wave of the user from a brain wave detector attached to 3D glasses which are interlocked with the 3D apparatus.

3. The method as claimed in claim 1 or claim 2, further comprising:
if a specific manipulation is input from a user, displaying a GUI for setting the display apparatus,
wherein the generating comprises generating a control signal for manipulating a setting of the GUI according to the obtained brain wave information.

4. The method as claimed in claim 3, wherein if the GUI is to adjust volume, the generating comprises generating a control signal to increase volume if it is determined that first brain wave information is included in the obtained brain wave information, and generating a control signal to decrease volume if it is determined that second brain wave information is included in the obtained brain wave information.

5. The method as claimed in claim 3, wherein if the GUI is to change channels, the generating comprises generating a control signal to change channels in a first direction if it is determined that third brain wave information is included in the obtained brain wave information, and generating a control signal to change channels in a direction opposite to the first direction if it is determined that fourth brain wave information is included in the obtained brain wave information.

6. The method as claimed in one of claim 1 to claim 5, wherein the pre-stored brain wave information is mapped with a control signal of the display apparatus and is stored separately for each of a plurality of users.

7. The method as claimed in one of claim 1 to claim 6, further comprising:
storing the obtained brain wave information; and
transmitting the stored brain wave information to an external health check-up system to perform health check-up of the user using a brain wave.

8. The method as claimed in one of claim 1 to claim 7, further comprising:
obtaining a body mass index using the obtained brain wave information; and
displaying the obtained body mass index.

9. The method as claimed in one of claim 1 to claim 8, further comprising:
controlling a selection and a volume of music of a meditation application provided by the display apparatus using the obtained brain wave information.

10. The method as claimed in one of claim 1 to claim 9, further comprising:
determining user preference of contents using the obtained brain wave information; and
providing recommended contents based on the determination results.

11. A display apparatus, comprising:
a display unit;
a storage unit which performs mapping brain wave information with a control signal of the display apparatus and stores the information;
a receiving unit which receives brain wave information of a user;
a comparison unit which compares the obtained brain wave information with brain wave information pre-stored in the storage unit; and
a control unit which generates a control signal corresponding to the brain wave information obtained based on the comparison result and controls a display apparatus according to the generated control signal.

12. The apparatus as claimed in claim 11, wherein the display apparatus is a 3D display apparatus,
wherein the receiving unit obtains a brain wave of the user from a brain wave detector attached to 3D glasses which are interlocked with the 3D display apparatus.

13. The apparatus as claimed in claim 11 or claim 12, further comprising:
a GUI generating unit which generates a GUI for setting the display apparatus if a specific manipulation is input from a user,
wherein the control unit generates a control signal for manipulating a setting of the GUI according to the obtained brain wave information.

14. The apparatus as claimed in claim 13, wherein if the GUI is to adjust volume, the control unit generates a control signal to increase volume if it is determined that first brain wave information is included in the obtained brain wave information, and generates a control signal to decrease volume if it is determined that second brain wave information is included in the obtained brain wave information,
wherein if the GUI is to change channels, the control unit generates a control signal to change channels in a first direction if it is determined that third brain wave information is included in the obtained brain wave information, and generates a control signal to change channels in a direction opposite to the first direction if it is determined that fourth brain wave information is included in the obtained brain wave information.

15. The apparatus as claimed in one of claim 11 to claim 14, wherein the pre-stored brain wave information is stored separately for each of a plurality of users.
